# EUROPEAN PATENT APPLICATION

(11) **EP 1 195 606 A1**
(43) Date of publication of application: **10.04.2002**
(21) Application number: 00890296.7
(22) Date of filing: 03.10.2000
(51) Int. Cl.: G01N 33/68

(54) **Allergen-microarray assay**

(71) Applicant: VBC-Genomics Forschungsges.m.b.H., 1070 Wien (AT)
(72) Inventor: Hiller, Reinhard Dr., 1120 Wien (AT); Harwanegg, Christian, 1100 Wien (AT); Müller, Manfred W., Prof. Dr., 3400 Klosterneuburg (AT)
(74) Representative: Alge, Daniel, Mag. Dr. rer.nat.

(57) **Abstract**

A method is provided for the detection of an immunoglobulin which binds to an allergen in a sample, whereby one or more allergens are immobilized on a microarray chip after which the sample is incubated with the immobilized allergens so that immunoglobulins which are specific for the allergens bind to the specific allergen after which the immunoglobulins which are bound to the specific immobilized allergens are detected, as well as a method for in vitro diagnosis of allergies in a patient.

## Description

The present invention relates to a method for the detection of an immunoglobulin which binds to an allergen in a sample as well as a method for in vitro diagnosis of allergies in a patient.

An allergy is a reaction produced by the body's immune system to a substance that would normally be thought of as harmless. It is this response that causes the symptoms that are classed as allergic reactions. Allergy is therefore not a failure of the immune system, but its over activity. The response of an allergic person to an allergen can produce a wide range of symptoms. Some people suffer symptoms such as asthma, eczema, rashes, itchy eyes, sinusitis, blocked or runny nose and hay fever, however, more serious symptoms can occur. With allergies such as those to venoms, nuts and shellfish, for example, a potentially life threatening condition called anaphylactic shock can occur. This happens when the body produces a reaction so severe that the throat swells, blood pressure drops and the person has difficulty in breathing. In some cases this type of reaction can be fatal.

The incidence of allergies is increasing in the developed countries (i.e. in Europe, Northern America and Japan). Estimates range from 20-50% of the population being affected. It is now known that allergies are the result of an unbalance in the T-cell compartment of the immune-system. More precisely, allergies are accompanied by an increased activity of so-called T-helper 2 (Th2) cells relative to T-helper 1 (Th1) cells, giving rise to increased IgE production.

IgE (immunoglobulin E) is at least one of the substances that cause allergic reactions. IgE specific for an allergen is not normally detected in the blood and is only produced when a person becomes sensitised to a substance. Substances that cause an allergy (called an allergen) produce a specific IgE that is unique to and will only react with it. This reaction between IgE and the allergen is like a lock and key. IgE, when combined with the allergen, causes cells to release chemicals (e.g. histamine), which cause the symptoms of allergy. A person may have specific IgE to more than one substance and may therefore be allergic to more than one substance. Results for IgE testing are expressed as a grade that indicates how much IgE specific to the substance tested for, is present in the blood. The higher the grade the more likely the patient is to be allergic. Over the past decades allergic diseases have been diagnosed using only scarcely purified extracts derived from the major allergen sources. Evidently, these crude mixtures are complex and variable in composition. As a consequence, the diagnostic potential of these extracts may vary and lead to the production of false-negative results. The latter has been ascribed mainly to the instability of allergens in extracts. Another drawback of using extracts in the diagnosis of allergies is the poor standardisation of commercially available products with respect to their allergenic composition. Units that express the potencies and methods of calculation are different for each company in the market. As a consequence, diagnostic products from different companies can hardly be compared.

The most important disease-related components, the major allergens, have been identified over the past decades, but their quantification is not used as the basis for standardisation of allergen extracts. Monoclonal antibodies against most major allergens are now available.

Allergy testing according to known methods is not a straightforward process. These methods can be useful procedures provided that a history is taken to identify which tests would be most appropriate. However, there are only very few recognised medical tests that can identify allergies and as a general rule these have been restricted to clinical laboratories or specialist centres. Whilst the diagnosis of atopy or allergy by a qualified practitioner or specialist in allergy can be relatively easily made further tests are often required to confirm this.

There are various types of allergy testing:
1. Skin Prick testing
   Suspected allergens are injected just under the surface of the skin and the reaction is observed by a qualified nurse or doctor. As the reaction develops there is a zone of redness, the stronger the reaction the greater the zone. Skin testing is quite sensitive although not all allergies can be identified by this method.
2. Patch testing
   Patch testing may be useful in cases of contact dermatitis. Test substances are usually applied to the skin covered by a patch and left in place for 48 hours. A positive reaction produces a small area of eczema. Again the reaction is observed by a qualified nurse or doctor.
3. Alternative allergy testing
   There are many alternative types of allergy testing and unfortunately few are accurate or recognised by the medical profession. These alternative tests are often misleading as well as costly.
4. Vega test
   The Vega test is an electrical test described as bioenergetic regulatory technique. The machine measures conductivity with a Wheatstone circuit. Electrodes are connected to acupuncture points or held in the patient's hand. Different solutions are then placed in a metallic tray. The machine is then calibrated by placing a glass vial containing a toxic substance into the tray. The vial causes a reduction in electrical conductivity. Other substances are then placed into the tray and if they give a similar reading this is reported as an allergic or "sensitive" reaction. This technique is widely used in health food stores however, its value is unproved and there are no valid trials that will substantiate claims.
5. Leucocytotoxic test
   The Leucocytotoxic involves mixing the patient's white blood cells with an extract of specific food and then measuring the cells in different ways for evidence of some form of change. There are a high number of false positive and false negative reactions and the American Academy of Allergy concluded that there was no evidence that the test was effective for the diagnosis of food or inhalant allergy.
6. Hair analysis
   Another form of testing is hair analysis. The hair can be analysed for the presence of toxic metals such as Lead, Mercury and Cadmium or low levels of Selenium, Zinc, Chromium, Manganese and Magnesium. Heavy metal poisoning is well recognised and documented in forensic science and hair analysis can indicate exposure to metals. However, hair analysis as a means to diagnosing allergy, through methods such as "dowsing", have never been validated.
7. Applied kinesiology
   Samples of food are placed under the tongue or held in a glass container in the hand of the patient. The patient is then asked to push his free arm against that of the examiner. If an allergic response is detected this manifests as a reduced muscle response and the patient experiences difficulty in raising his arm. This technique fails to with stand up against a double blind study.
   Furthermore, there are various in vitro methods for diagnosing allergies which detect the presence of IgE or IgG antibodies in the blood of a patient:
8. Conventional methods as ELISA or Western blots are used to detect antibodies (IgE) present in serum of a patient with the help of (recombinant) allergens.
9. Radioallergosorbent test (RAST®) :
   In this procedure a specific allergen is coupled to a paper disk; immunospecific IgE, if present in the test (patient's) serum will bind to the disk; detection is effected by radiolabeled anti-IgE. Different scoring systems comparing test results with the absolute binding of a negative control are in use. Commonly, the modified RAST® procedure is followed with overnight incubation and results in higher sensitivity.
10. RAST based quantitative IgE inhibition experiments in which allergen extract is dotted on nitro-cellulose strips. Aliquots of sera are preincubated with a mixture of specific recombinant allergens after which this mixture is incubated on the nitro-cellulose strips. Bound IgE is detected with anti human IgE antibodies. The percentage inhibition of IgE binding the natural extracts after preabsorption with recombinant allergens is calculated in a last step.
11. Cellular antigens stimulation test (CAST) according to which patient basophilic cells are stimulated with interleukin-3 and allergen followed by measurement in an ELISA of de novo generated and released sulfidoleukotriene (SLT).
12. UniCAP: Recombinant allergens are immobilized on a solid phase structure after which the binding of antibodies which are present in the serum of a patient to the allergens is detected.

However, these methods require a multitude of individual experimental steps in order to test a larger number (e.g. 100) different allergens. Furthermore, these above mentioned tests do not show a sufficiently high sensitivity and reliability and are very time consuming. Also the amounts of sample (e.g. serum of a patient) as well as purified, possibly recombinant, costly allergens which are needed to carry out these tests are large.

The object of the present invention is therefore to provide a method for the detection of an immunoglobulin which binds to an allergen in a sample which does not show the above mentioned drawbacks and which can be carried out in a short time with only a small amount of sample and allergens, which is highly reliable and sensitive and most importantly allows the detection of a practically unlimited number of allergens in one single assay.

A further object of the present invention is to provide a method for in vitro diagnosis of allergies in a patient without the above mentioned drawbacks.

The above mentioned method is characterized in that one or more allergens are immobilized on a microarray chip after which the sample is incubated with the immobilized allergens so that immunoglobulins which are specific for the allergens bind to the specific allergen after which the immunoglobulins which are bound to the specific immobilized allergens are detected.

Microarrays have been adopted recently for a number of DNA manipulating techniques that are established in the scientific community since long. These DNA chips have become available in a number of different formats and will eventually change ways of designing experiments in the ordinary laboratory work. In vitro DNA diagnosis has become less time-consuming and labor-spending since this novel technology allows assay complexity in a high-throughput format. Consequently, in the area of proteome research, classic solid phase substrates, such as microtiter plates, membrane filters and microscopic slides are being turned into the high-density microarray format. The new and versatile protein array technology eventually allows high-throughput screening for gene expression and molecular interactions (Walter et al., Curr Opin Microbiol 2000 Jun;3(3):298-302; Emili & Cagney, Nat Biotechnol 2000 Apr;18(4)393-7). Recently, the concept of protein arrays has been adopted for its use in immunological assays.

A biochip is described as capable of supporting high-throughput (HT), multiplexed enzyme-linked immunosorbent assays (ELISAs). These biochips may consist for example of an optically flat glass plate containing 96 wells formed by an enclosing hydrophobic Teflon mask, however, also other materials and forms of biochips are known and used. Experiments demonstrate that specific multiplex detection of protein antigens arrayed on a glass substrate is feasible. Further application of this new high-throughput screening (HTS) format include direct cellular protein expression profiling, multiplexed assays for detection of infectious agents and cancer diagnostics (Mendoza LG et al, Biotechniques 1999 Oct;27(4):778-80).

However, in these known microarray chip methods testing proteins the antibodies are immobilized to the microarray chip. Surprisingly, it has been found that it is possible not only to bind the antibodies to the microarray chip but even allergens which are the antigens corresponding to specific antibodies, in particular IgE and IgG, respectively. This fact is particularly surprising since functional allergens show a particular secondary structure which may be modified when bound in such high density to a solid phase as the microarray chip. This modification of the structure of the allergen would interfere with the antibody-allergen binding which would lead to false negative results. This is not the case with the immobilization of antibodies, fragments of antibodies or peptides, since they are relatively small and show high stability.

Moreover, diversity of possible allergens is of course much higher than antibody diversities with respect to handling the proteins, especially for immobilizing and staining such a series of structurally diverse allergens. However, it has been surprisingly shown that allergens immobilized on a microarray chip show high reliability and sensitivity in a method for the detection of immunoglobulins in a sample.

This method allows to carry out an assay for a multitude of immunoglobulins which bind to a specific allergen in one experimental step which assay can also be atomised: More than 100 different allergens can be tested without having to carry out a multitude of individual experimental steps as when performed in a conventional microtiter form. Furthermore, this assay shows a high degree of sensitivity and reliability. Also, the test results of this method can be obtained within a short period of time, e.g. about three hours, shortening the assay time when compared to conventional RAST or ELISA tests.

Furthermore, the microarray assay is highly reproducible when performing repetitive experiments with chips containing samples from different persons, e.g. by a microtiter plate-like mask whereby every well comprises a number of spots of different allergens and a sample of one person is added per well. A further advantage of this method according to the present invention is due to the fact that a large number of different probes occupies a relatively small area providing a high density. The small surface area of the array permits extremely uniform binding conditions (temperature regulation, salt content, etc.) while the extremely large number of probes allows massively parallel processing of hybridizations. Because the high density arrays contain such a large number of probes it is possible to provide numerous controls including, for example, controls for variations or mutations in a particular allergen, controls for overall hybridization conditions, controls for sample preparation conditions, and mismatch controls for non-specific binding or cross hybridization.

Further, the assay requires only a minimal fraction of material, (allergen as well as a sample) when compared to conventional test systems. This means that with an equal amount of starting material a much greater number of test kits can be manufactured when using the microarray form and a smaller amount of sample can be used to test for a larger amount of immunoglobulins which bind to allergens. Also, in small volumes, binding may proceed very rapidly.

Natural intact "immunoglobulins" or antibodies comprise a generally Y-shaped tetrameric molecule having an antigen binding-site at the end of each upper arm. An antigen binding site consists of the variable domain of a heavy chain associated with the variable domain of a light chain. More specifically, the antigen binding site of an antibody is essentially formed by the 3 CDRs (complementarity determining regions) of the variable domain of a heavy chain (V.sub.H) and the 3 CDRs of the variable domain of the light chain (V.sub.L).

Generally the term "antigen" refers to a substance capable of eliciting an immune response and ordinarily this is also the substance used for detection of the corresponding antibodies by one of the many in vitro and in vivo immunological procedures available for the demonstration of antigen-antibody interactions.

Similarly, the term "allergen" is used to denote an antigen having the capacity to induce and combine with specific (i.e., IgE) antibodies which are responsible for common allergies; however, this latter definition does not exclude the possibility that allergens may also induce reactive antibodies, which may include immunoglobulins of classes other than IgE.

"Immobilizing" in the context of the proteins or peptides refers to the binding or attaching of the protein/peptide to solid supports by conventional means, with or without an additional spacer between the solid support and the allergen. Immobilizing peptides/proteins to a support is well known in the art; in the scope of the present invention any immobilization is comprised, e.g. covalent, non-covalent, in particular by hydrophobic interactions with for example membranes and synthetic surfaces, respectively, etc.

A "microarray" is an array of features (e.g. "spots") having a density of discrete features of at least about 16/cm², preferably at least about 64/cm², still preferred about 96/cm² and most preferred at least about 1000/cm². The features in a microarray have typical dimensions, e.g., diameters, in the range of between about 10-2000 µm, preferably about 50-500 µm, still preferred about 150-250 µm, and are separated from other features in the array by about the same distance. Therefore, a microarray according to the present invention useable for routine mass screening may have at least 500, preferably at least 1000 individual spots comprising allergens, standards and controls.

The "chip" according to the present invention may be of virtually any shape, e.g. a slide or a well of a microtiter plate, or even a multitude of surfaces although a planar array surface is preferred.

The detection step can be carried out with any conventional method known by the person skilled in the art e.g. by physical, enzymatic, chemical reaction, etc.

According to a preferred embodiment of the present invention IgEs are detected as immunoglobulins. IgE is known as a substance that causes allergic reactions and is only produced when a person becomes sensitive to a substance, an allergy. Therefore, in order to test if the sample is from a patient who is allergic to the specific allergen the sample is tested for IgE as immunoglobulins. The higher the amount of IgE in the sample the more likely the patient the sample is taken from is allergic to the specific allergen.

Preferably IgG are detected as immunoglobulins. IgG is known to be present in a sample of a patient who is allergic to food, therefore the detection of IgG can be used as an indication for food intolerance.

Advantageously one or more native allergens are immobilized on the microarray chip. These - single - allergens are for example purified from an allergen extract, e.g. from a specific food or plant in this case. One or more allergens of a specific species can be analysed at once. The methods of purification are in principle known to the person skilled in the art, e.g. chromatographic purification, purification by mass separation, purification by specifically binding the allergen to a solid phase, e.g. over an antibody, etc., but application of highly purified allergens for mass production of allergy testing has not yet been proposed.

Preferably one or more recombinant allergens are immobilized on the microarray chip. Over the last few years a number of recombinant allergens have been produced. The production of recombinant allergens is also in principle known state of the art but had little impact on routine allergy testing. By using recombinant allergens it is possible to provide a great number of one or more specific allergens. It is furthermore possible to modify the recombinant allergens specifically in order to produce any allergen mutations to detect for specific immunoglobulins. Furthermore, making use of the major allergens as recombinant proteins provides an alternative to the extract based tests with respect to assay stability as well as to diagnostic standardisation. Recombinant (or synthetic) allergens may also be better to standardize with respect to their production method. Moreover, differences between production lots are smaller for recombinant (or synthetic) allergens than for allergens derived from natural sources. It has been surprisingly shown with the present invention that the use of these recombinant allergens in the routine in vitro diagnostic tests according to the present invention is equal or superior to conventional extract based test systems. In contrast to the routine serial testing according to the state of the art, the present allergen testing using also recombinant allergens brings a completely new quality with respect to standardisation, controllability, reproducibility, etc. for standard allergen testing.

Recombinant allergens are for example those described in the publications by Chapman et al. Allergy, 52:374-379 (1997), Laffer et al. J Allergy Clin Immunol Vol 98 Number 2 652-658 (1996), Müller et al. Clinical and Experimental Allergy Vol 27 9. 915-920 (1997), Niederberger et al. J Allergy Clin Immunol Vol 102 Number 4, Part 1 579-591 (1998), Menz et al. Clinical and Experimental Allergy Vol 26, 50-60 (1996) which are incorporated herein by reference. Further examples of (recombinant) allergens are but not limited to rBetv1, rBetv2, rBetv4, rJunO2, Cass1, rPhlp1, rPhlp2, rPhlp4, rPhlp5a, rPhlp6, rPhlp7, rPhlp11, rPhlp12, rParj2, Artv1a, Mugwort profilin, Apig1, Apig1.0201, Dauc1.2, rArah2, rArah5, Mald1, Mald2, rPena1, recCarp, rDerp1, rDerp2.0101, rDerp2, rDerp2b, rDerp5, rDerp5a, rDerp7, rDerp8, rDerp10, rTyrp2, rLepd2.01, rLepd13, rEurm2.0101, rFeld1, rFeld1a, rBosd2, a representative allergen from Cat, a representative allergen from Dog, a representative allergen from BSA, a representative allergen from Mouse, a representative allergen from Rat, a representative allergen from Pig, a representative allergen from Sheep, a representative allergen from Chicken, a representative allergen from Rabbit, a representative allergen from Hamster, a representative allergen from Horse, a representative allergen from Pigeon, a representative allergen from Guineaalbumin, HSA, a-NAC, rPenc3, Penn13, rPenc19a, rPenc19b, rAspf1, rAspf1a, rAspf3, rAspf3a, rAspf4, rAspf4a, rAspf6, rAspf6a, rAspf7, rAspf8, rAlta1, rAlta2, rMalf1, rMalf5, rMalf6, rMalf7, rMalf8, rMalf9, rHevb1, rHevb1a, rHevb3, rHevb8, rHevb9, rHevb10, rHevb11, rAK, rBlag2, rBlag4, rBlag5, Phospholipase A, Hyaluronidase, rVesv5, rVesg5.

According to a further advantageous embodiment one or more synthetically produced allergens are immobilized on the microarray chip. The method for synthetically producing peptides or proteins is well known in the state of the art; by synthetically producing allergens it is possible to provide highly purified allergens in a great number and at low costs since such production methods are highly automatized. Furthermore, the exact sequence of any allergen can be provided with or without modifications, which increases the sensitivity and reliability of the method for detecting the immunoglobulins. It is also possible to use only the allergenic determinant or the allergenic domain of a specific allergen in the test according to the present invention. This may eliminate the risks and drawbacks of working with large proteins, because shorter peptidic structures are easier to handle for routine production of biochips.

It is of particular advantage to provide the native form of the allergen as well as a synthetic or recombinant form of the allergen on the same chip. Whereas it is preferred to use mainly recombinant or synthetic allergens. The native form of an allergen may be provided at least as a control or additional quality information on the chip. A preferred chip according to the present invention therefore comprises a native and a synthetic or recombinant form of at least one allergen. This provides also for improved quality control and standardisation of different batches of allergens.

Preferably one or more haptens are immobilized as allergens on the microarray chip. A "hapten" is a low molecular weight (typically weighing less than about 7000 Daltons) substance that is generally incapable of causing, by itself, a significant production of antibodies upon administration to an animal body, including a human body. This can occur because a hapten is too small to be recognized by the body's immune system. However, when a hapten is coupled to a larger, carrier molecule, the hapten can acquire antigenic properties. In other words, binding the hapten to the carrier molecule (to make an analyte-carrier molecule combination) permits the bound hapten to be recognized by an animal's immune system. An immunoprecipitation reaction can take place between the hapten (coupled to the carrier molecule), and an antibody to the hapten. By providing haptens which are immobilized to the microarray chip a higher density can be achieved on the chip.

It is of course possible to combine these above mentioned different forms of allergens, e.g. to use at the same time recombinant and (purified) native allergens. The combination of these different forms allows to compare them and carry out a control of the e.g. recombinant allergens but also as a control for the various native lots of the allergens which may vary due to differences in biological sources, methods of preparation, purity, etc.

For a highly efficient test it is preferable to use allergens which show optimal features, e.g. a high binding capacity. However, the use of less or differently active antigens is preferred for the detection of inefficiently binding allergen-variants, e.g. for the use in hyposensitization therapies.

According to a preferred embodiment of the present invention the allergens are immobilized on a 10 to 2000 µm diameter, preferably 50-500 µm diameter, still preferred 150-250 µm diameter, spot on the microarray chip. Since the spots have a small diameter, it is possible to immobilize a great amount of different allergens and various concentrations of specific allergens on separate spots of the microarray chip, thus allowing to provide one microarray chip which can in one - automatized - step be used to analyse a big number of allergens or allergen concentrations.

Preferably the allergens are immobilized on a solid support, preferably a glass carrier, synthetic carrier, silicon wafer and membrane, respectively. Such chips can be produced for example according to methods described in Ge, H. (2000), Nucleic Acids Research, 28, e3 (i-vii); Qian W. et al. (2000), Clinical Chemistry, 46 (1456-1463); MacBeath G. et al. (2000), Science, 289, (1760-1763), which are disclosed herein by reference. Each of these materials shows specific characteristics and advantages which are well-known to the person skilled in the art. Therefore, the material for the microarray chip will be chosen according to the allergen which is to be tested, the method for the detection of bound immunoglobulins, the used buffers. Furthermore, the choice of material is also a financial question.

Preferably the microarray chip is chemically modified, preferably by aminoreactive and carboxyreactive modification, respectively. This allows to precisely determine the way the allergen is bound to the microarray chip.

Advantageously the allergens are covalently bound to the microarray chip. This provides a stable binding of the allergens to the microarray chip, thereby providing a method which is particularly reliable.

According to another advantageous embodiment of the present invention the immunoglobulins are detected in blood serum as the sample. In patients who are allergic to an allergen immunoglobulins are produced mainly in the blood serum, so that analyzing the blood serum provides a reliable method for detecting immunoglobulins. Furthermore, blood serum can easily be collected from the patient in a very simple way and the collection of the sample can be carried out even at the home of the patient.

Preferably the blood serum is diluted 1:1 - 1:15, preferably 1:5. The dilution can be carried out with any suitable solution, e.g. 1x TBST (10 mM Tris-HCl, pH 8,0, 150 mM NaCl, 0,5% TWEEN 20)

Advantageously the sample is incubated 1 min. to 24 hours, preferably 1 - 2 hours, with the allergens. Of course, it is possible to incubate the sample with the allergens even over a period of days. However, this does not result in an increase in signal intensity or specificity. In general, an incubation time of 1 hour is sufficient for a reliable and sensitive result.

Furthermore, the sample is preferably incubated at a temperature between 0 and 60°C, preferably at 37°C, with the allergens. Incubations at lower temperatures do not result in an increase of signal, but they rather lead to an increase in unspecific binding and background noise. An incubation at 37°C has shown to provide exact and reliable results for allergen test in humans.

Advantageously the bound immunoglobulin is detected with at least one labelled specific anti-immunoglobulin antibody. As used herein, the term "antibody" refers to intact molecules as well as fragments thereof, such as Fa, F(ab).sub.2, and Fv, which are capable of binding the epitopic determinant. Antibodies can be prepared using intact polypeptides or fragments containing small peptides of interest as the immunising antigen. The polypeptide or peptide used to immunise an animal can be derived from the transition of RNA or synthesized chemically, and can be conjugated to a carrier protein, if desired. Commonly used carriers that are chemically coupled to peptides include bovine serum albumin and thyroglobulin. The coupled peptide is then used to immunise the animal (e.g., a mouse, a rat, or a rabbit).

In the scope of the present invention the term antibody refers to monoclonal or polyclonal antibodies whereby monoclonal antibodies may be prepared using any technique which provides for the production of antibody molecules by continuos celllines in culture. These include but are not limited to the hybridoma technique, the human B-cell hybridoma technique and the IBV hybridoma technique. Furthermore, chimeric antibodies may be produced, single chain antibodies as well as synthetically produced antibodies.

The antibody may be labelled according to any known method which allows to qualify and preferably quantify the bound antibody. Detectable labels suitable for use in the present invention include any composition detectable by spectroscopic, photochemical, biochemical, immunochemical, electrical, optical or chemical means. Useful labels include biotin for staining with labelled streptavidin conjugate, magnetic beads, fluorescent dyes (e.g. fluorescein, texas red, rhodamine, green fluorescent protein, and the like), radiolabels (e.g. ³H, ¹²⁵I, ³⁵S, ¹⁴C or ³²P), enzymes (e.g. horse radish peroxidase, alkaline phosphatase and others commonly used in ELISA), and colorimetric labels such as colloidal gold or coloured glass or plastic (e.g. polystyrene, polypropylene latex, etc.) beads.

Means of detecting such labels are well known to those of skill in the art. Thus, for example, radiolabels may be detected using photographic film or scintillation counters, fluorescent labels may be detected using a photodetector to detect emitted light. Enzymatic labels are typically detected by providing the enzyme with a substrate and detecting the reaction product produced by the action of the enzyme on the substrate, and colorimetric labels are detected by simply visualising the coloured label.

Preferably the bound immunoglobulins are detected with at least one fluorescence and radioactive, respectively, labelled specific anti-immunoglobulin antibody. These are classic methods for qualitatively and quantitatively analyzing bound antibody which are very specific and reliable.

Other advantageous detection systems for micro arrays are for example those described in Schult K. et al. (1999), Anal Chem, 71 (5430-5435); Vo-Dinh T. et al. (1999), Anal Chem, 71 (358-363); Brignac SJ Jr. et al. (1999), IEEE Eng Med Biol Mag, 18 (120-122); Otamiri M. et al. (1999), Int J Biol Macromol, 26 (263-268); Wright, GL Jr. et al. (2000), Prostate Cancer and Prostatic Diseases, 2 (264-276); Nelson RW. et al. (2000), Electrophoresis 21 (1155-1163); Rich RL. et al. (2000), Curr Opin Biotechnol 11 (54-61); Chen JJ. et al. (1998), Genomics, 51 (313-324), which publications are enclosed herein by reference.

Advantageously one or more indoor allergens are immobilized as allergens. These may be but are not limited to Mites, Tyr. put, Lep. dest. or .mayrei, Felis, Bos, Albumine, Pen. cit., Pen. not., Asp. fumigatus, Alt. alt., Malassezia furfur, Latex, Plodia, Blatella.

According to a further preferred embodiment one or more outdoor allergens are immobilized as allergens. These may be but are not limited to Betula, Juniperus, Phleum, Parietaria judicea.

Further one ore more food allergens may be immobilized as allergens. Examples are sellerie, karott, peanut, apple, shrimp, fish.

Further one ore more venom allergies are immobilized as allergens. These may be but are not limited to Bee or Wasp.

Also one or more auto-allergens may be immobilized as allergens. Such auto-allergens may be e.g. liver membrane antigens, ssDNA antigens, antigens in or on skeleton muscle cells, etc.

A further aspect of the present invention relates to a method for in vitro diagnosis of allergies in a patient, whereby a serum sample is taken from the patient after which the sample is analysed for immunoglobulins which bind to allergens according to an above mentioned method according to the present invention, whereby a microarray chip is used on which at least 10, preferably at least 50, still preferred at least 90, different allergens are immobilized, after which a positive reaction between the sample and the immobilized allergens is diagnosed as an allergy. The above mentioned definitions and advantages relate also to this method for in vitro diagnosis of allergies compared to the known methods for diagnosing allergies. The present method shows the advantage that a big amount of allergies can be simultaneously diagnosed with only one sample since all the allergens which are to be tested are immobilized on one single microarray chip. Every step is therefore carried out only on one chip, whereby the chip further may comprise spots with no immobilized allergens and therefore allowing simultaneous negative detection. The amount of allergens to be tested is not limited and it is of course possible to provide two or more microarray chips.

A further aspect of the present invention relates to a microarray chip on which one or more allergens are immobilized. Also in this aspect the above mentioned definitions and advantages are applied.

Preferably the allergens are immobilized on a 100 to 500 µm diameter, preferably to 200-300 µm diameter, spot.

Still preferred the microarray chip is a glass carrier, synthetic carrier, silicon wafer and a membrane, respectively.

Advantageously the microarray chip is chemically modified, preferably by aminoreactive and carboxyreactive modification, respectively.

Advantageously the allergens are covalently bound to the microarray chip.

A further aspect according to the present invention relates to a kit which comprises a microarray chip according to the present invention as mentioned above and a first reagent comprising at least one immunoglobulin detecting reagent, preferably an anti-immunoglobulin antibody, preferably in a known concentration, and possibly a second reagent as a positive sample comprising at least one immunoglobulin which binds to an allergen. The first reagent comprising the at least one immunoglobulin detecting reagent, preferably an anti-immunoglobulin antibody may be used for the detection of bound immunoglobulin in which case the anti-immunoglobulin antibody is preferably labelled as mentioned above. Preferably an antibody is present in the first reagent in a known concentration thereby allowing to provide for reproducible results. Furthermore, the kit preferably comprises a second reagent as a positive sample comprising at least one immunoglobulin which binds to an allergen. Also here it is preferable that the immunoglobulin is present in a known concentration in the second reagent thereby allowing to quantify the immunoglobulin present in the sample by comparing the results of the sample of the patient with the results of positive sample (the second reagent).

Preferably the kit is provided for carrying out a method according to the present invention as mentioned above. For this the first and second reagents may be designed in order to detect one specific immunoglobulin which binds to a specific allergen or one specific allergy in a patient. However, the kit may also be designed to detect a variety of immunoglobulins which bind to specific allergens or to diagnose a variety of allergies in a patient.

The present invention is now described in more detail with the following examples and figures to which it is of course not limited.
Fig. 1 shows a layout of an allergen microarray;
Fig. 2 shows a scan image of an allergen array assayed with serum from an allergic person;
Fig. 3a-3c show a Scatterplot Test from a triple assay.

### Example 1 :

### Allergen spotting

Allergens were arrayed using a GMS 417 spotter from Genetic Microsystems. The proteins were spotted on derivatized glass slides. Individual allergens were spotted at one hit per dot as triplicates. The dots (features) showed a diameter of about 200 µm.

The allergens were assembled in functional groups as follows:
(A) Outdoor (I. Trees [1=rBetv1, 2=rBetv2, 3=rBetv4, 4=rJunO2, 5=Cass1], II. Grasses [6=rPhlp2, 7=rPhlp4, 8=rPhlp5a, 9=rPhlp1, 10=rPhlp6, 11=rPhlp7, 12=rPhlp11, 13=rPhlp12], III. Weeds [14=rParj2, 15=Artv1a, 16=Mugwort profilin]),
(C) Food Allergens (X. Vegetables [17=Apig1, 18=Apig1.0201, 19=Dauc1.2, 20=rArah2, 21=rArah5], XI. Fruits [22=Mald1, 23=Mald2], XII. Shrimps [24=rPena1], XIII. Fish [25=recCarp]),
(B) Indoor (IV. Mites [26=rDerp1, 27=rDerp2.0101, 28=rDerp2, 29=rDerp2b, 30=rDerp5, 31=rDerp5a, 32=rDerp7, 33=rDerp8, 34=rDerp10, 35=rTyrp2], V. Animals [36=rLepd2.01, 37=rLepd13, 38=rEurm2.0101, 39=rFeld1, 40=rFeld1a, 41=rBosd2, 42=a representative allergen from cat, 43=a representative allergen from dog, 44=BSA, 45=a representative allergen from mouse, 46=a representative allergen from rat, 47=a representative allergen from pig, 48=a representative allergen from sheep, 49=a representative allergen from chicken, 50=a representative allergen from rabbit, 51=a representative allergen from hamster, 52=a representative allergen from horse, 53=a representative allergen from pigeon, 54=guineaalbumin], VI. Moulds [55=rPenc3, 56=rPenc19a, 57=rPenc19b, 58=Penn13, 59=rAspf1, 60=rAspf3, 61=rAspf4, 62=rAspf6, 63=rAspf1a, 64=rAspf3a, 65=rAspf4a, 66=rAspf6a, 67=rAspf7, 68=rAspf8, 69=rAlta1, 70=rAlta2], VII. Yeast [71=rMalf7, 72=rMalf1, 73=rMalf5, 74=rMalf6, 75=rMalf8, 76=rMalf9], VIII. Latex [77=rHevb1, 78=rHevb1a, 79=rHevb3, 80=rHevb8, 81=rHevb9, 82=rHevb10, 83=rHevb11], IX. Insects [84=rAK, 85=rBlag2, 86=rBlag4, 87=rBlag5]),
(D) Venoms (XIV. Bee [88=Ag5, 89=Phospholipase A, 90=Hyaluronidase, 91=rVesv5, 92=rVesg5], XV. Wasp [93=Ag5]),
(E) Auto-allergens (94=HSA, 95=a-NAC)

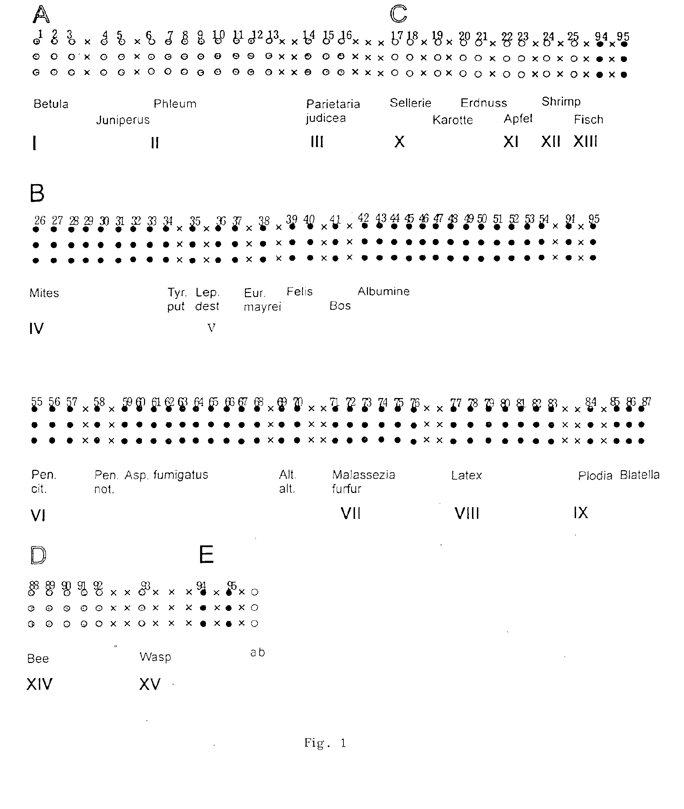

Additionally, buffer dots were interspersed between the allergen subgroups serving as a background control, marked as "X", "ab" is the labelled antibody. (Fig. 1: 1864x1182 pixels, 1,86x1,18 cm, 1000 pixels per cm, pixel depth/colours 8/256, 1 pixel corresponding to 10 µm).

100 µl aliquots of the allergens were divided into the wells of a 96 well microtiter plate at a concentration of ~200 ng/µl in spotting buffer (300 mM Sodium-phosphate pH 8.5). The optimal concentration for immobilization of the allergens was calculated from titration experiments with several allergens in different buffer solutions as well as on different slides. The proteins assayed displayed a saturation behaviour at concentrations equal to or larger than 100 ng/µl as became evident from a constantly strong white signal when scanning with a GMS scanner. At this concentration the binding behaviour of the allergens was independent of the composition of the storage as well as the spotting buffer.

### Example 2:

### SOPHIA (Solid phase Immunosorbent Assay)

Following over night incubation slides either purchased from CEL Associates (aldehyde slides) or prepared in-house were washed in 1x TBST (10 mM Tris pH 8.0/150 mM NaCl/0.5% Tween 20) under vigorous shaking in a Falcon tube at ambient temperature. As a blocking step, the slides were transferred to a 1x TBST solution containing 0.01% BSA for 2 hours at ambient temperature. Successively, slides were washed in 1x TBST for 15 min. and rinsed in distilled water briefly.

The allergen array was incubated with diluted serum (1:5 in 1x TBST) for (at least) 60 min. at 37°C with shaking. Various degrees of serum dilution have been tested, ranging from 1:1 - 1:15. Usually a dilution of 1:5 gave the best results. 30 µl of diluted serum was added to the slides in Press Seal Chambers purchased from SIGMA Technologies. The chambers were used as according to the manufacturers protocol. Various incubation times for the serum ranging from 60 min. to over night were assayed. However, an extended incubation with serum did not result in an increase in signal intensity or specificity. Following the incubation with serum slides were washed in 1x TBST (15 min., ambient temperature) with shaking.

Five different serums of allergic patients that have been examined using conventional diagnostic tests (skin prick test, RAST, ELISA) and a serum of a non-atopic patient were chosen adequate for a benchmark test of the allergen array. The individual sera were assayed at least twice on different batch produced allergen arrays.

Fluorescence-labelled α IgE antibody labelled with AlexaFluor fluorescence dye according to the manufacturer's protocol (Molecular Probes) was added to the allergen array in a solution containing 0.01% BSA/1x TBST and incubated for 60 min. at ambient temperature. Working dilutions for the antibody ranged from 1:1000 - 1:5000 depending on the time and efficiency of labelling. Following the immunoassay, slides were washed with 1x TBST (15 min., ambient temperature, shaking) and briefly rinsed with distilled water.

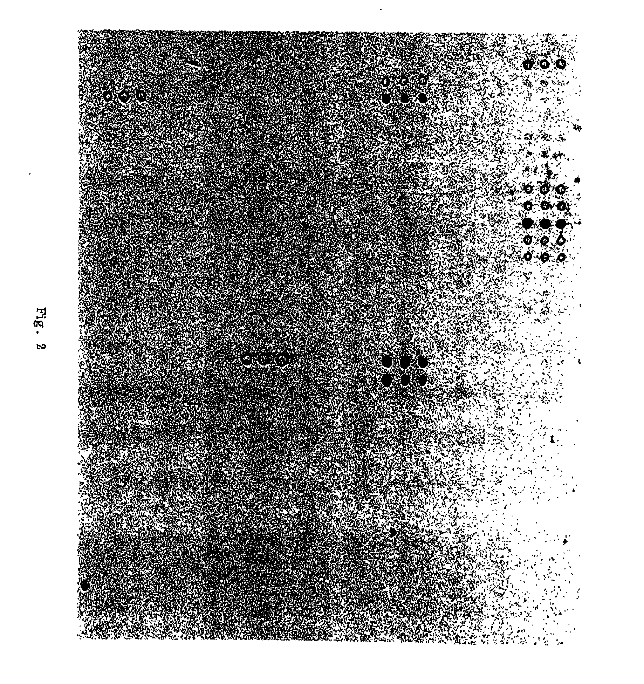

Following the immunosorbent assay, the slides were evaluated with a GMS two-colour scanner. Generally, signal intensities varied only slightly between different assays and different slides. However, the background values differed depending on the type of slides used. An example of a scanned image of an allergen array assayed with serum of a patient suffering of allergy is depicted in Fig. 2. This patient shows a strong reaction with Phleum, Mite, Felis and bee (cf. Fig. 1). No background signals stemming from an unspecific interaction with buffer dots or auto-allergens were observed. Following the complete evaluation of the allergic patient's sera the results were compared with data preliminary obtained for the identical sera using RAST tests. The data achieved with the method according to the present invention were in good agreement with those data sets available

### Example 3 :

### Reproducibility test assaying serum of patient C.

Serum of patient C was assayed three times on individually prepared allergen-microarrays. The experimental procedure was as essentially described before.

Following the assay, the slides were scanned using identical hardware settings. Data analysis was performed using the *GenePix* software package. The calculated mean values of the signal intensities for each allergen triplicate were compared after three repeats of the experiment.

Only values corresponding to signals that were at least 1.5 x higher than the mean value of the buffer spot signals were chosen for the final analysis. The mean value, standard deviation and percentage of standard deviation for the relevant signals are depicted in table 1.

**table 1**

| **Allergen** | **Test 1** | **Test 2** | **Test 3** | **Mean Value** | **Standard Dev.** | **% Mean** |
|---|---|---|---|---|---|---|
| **rBet v 1** | 20731 | 21325 | 25060 | 22372,00 | 1916,11 | **8,56** |
| **rPhl p 2** | 17176 | 15529 | 14227 | 15644,00 | 1206,67 | **7,71** |
| **rPhl p 4** | 36134 | 33511 | 29328 | 32991,00 | 2802,76 | **8,50** |
| **rPhl p 5a** | 56600 | 53068 | 55594 | 55087,33 | 1485,77 | **2,70** |
| **rPhl p 6** | 56244 | 51359 | 37625 | 48409,33 | 7882,14 | **16,28** |
| **rPhl p 12** | 6291 | 7003 | 12289 | 8527,67 | 2675,50 | **31,37** |
| **rPar j 2** | 7552 | 7678 | 9444 | 8224,67 | 863,73 | **10,50** |
| **Art v 1a** | 6997 | 7931 | 11258 | 8728,67 | 1828,70 | **20,95** |
| **Mugwort profilin** | 7901 | 7669 | 9260 | 8276,67 | 701,74 | **8,48** |
| **Mal d 1** | 9890 | 15295 | 8176 | 11120,33 | 3033,74 | **27,28** |
| **HSA** | 9868 | 8732 | 10708 | 9769,33 | 809,71 | **8,29** |
| **Sheepalbumin** | 9858 | 8295 | 10222 | 9458,33 | 835,92 | **8,84** |
| **Horsealbumin** | 9602 | 9061 | 10559 | 9740,67 | 619,37 | **6,36** |
| **rAsp f 1 (b)** | 12216 | 10129 | 10781 | 11042,00 | 871,77 | **7,90** |
| **rMal f 5** | 13459 | 11018 | 12035 | 12170,67 | 1001,14 | **8,23** |
| **rAK** | 20680 | 14202 | 19978 | 18286,67 | 2902,48 | **15,87** |

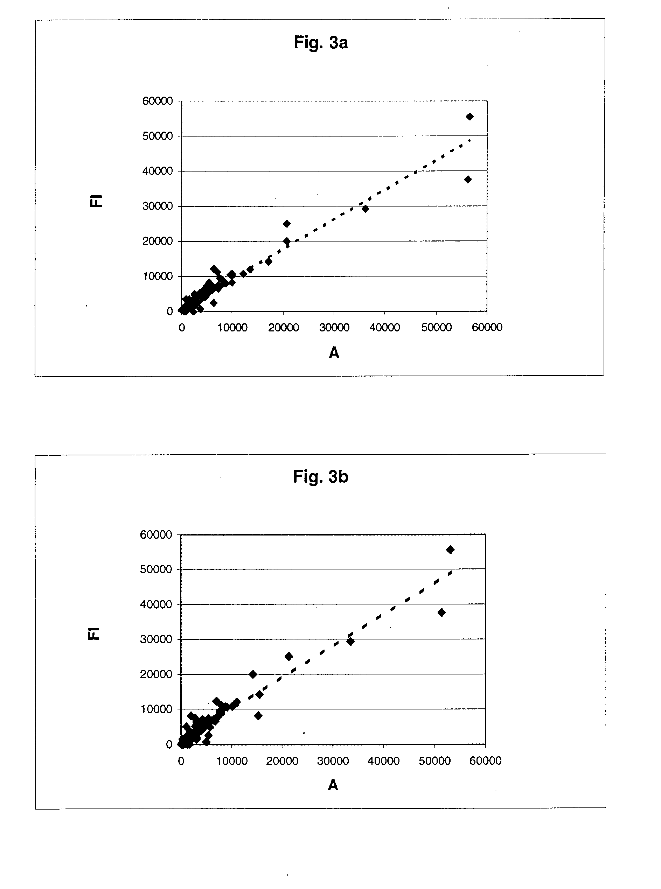

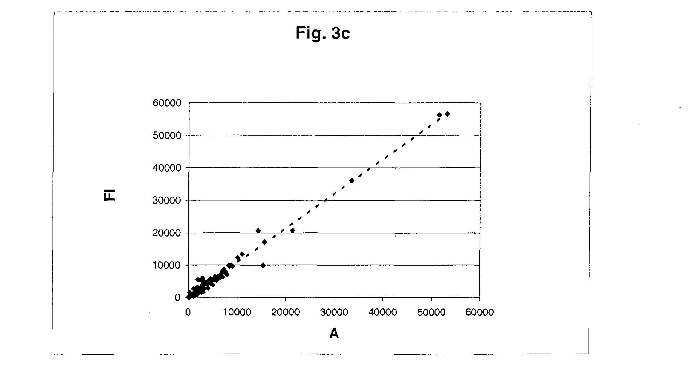

The mean standard deviation calculated from the values obtained after three individual assays was 12.36 %. This means that the chip-based immunological assay, questioning the presence of IgE in patients' sera, is highly reproducible. This is also evident when inspecting scatter-plots derived from the triple-assay 3, s. Fig. 3a-3c, wherein Fig. 3a shows a Scatterplot Test 1 vs. Test 3, Fig. 3b a Scatterplot Test 2 vs. Test 3 and Fig. 3c a Scatterplot Test 1 vs. Test 2; A showing the allergens and FI the fluorescence intensity.

## Claims

1. A method for the detection of an immunoglobulin which binds to an allergen in a sample, **characterized in that** one or more allergens are immobilized on a microarray chip after which the sample is incubated with the immobilized allergens so that immunoglobulins which are specific for the allergens bind to the specific allergen after which the immunoglobulins which are bound to the specific immobilized allergens are detected.

2. The method according to claim 1, **characterized in that** IgE are detected as immunoglobulins.

3. The method according to claim 1, **characterized in that** IgG are detected as immunoglobulins.

4. The method according to any one of claims 1 to 3, **characterized in that** one or more native allergens are immobilized on the microarray chip.

5. The method according to any one of claims 1 to 4, **characterized in that** one or more recombinant allergens are immobilized on the microarray chip.

6. The method according to any one of claims 1 to 5, **characterized in that** one or more synthetically produced allergens are immobilized on the microarray chip.

7. The method according to any one of claims 1 to 6, **characterized in that** one or more haptens are immobilized as allergens on the microarray chip.

8. The method according to any one of claims 1 to 7, **characterized in that** the allergens are immobilized on a 10 to 2000 µm diameter, preferably 50-500 µm diameter, still preferred 150-250 µm diameter, spot on the microarray chip.

9. The method according to any one of claims 1 to 8, **characterized in that** the allergens are immobilized on a solid support as the microarray chip.

10. The method according to any one of claims 1 to 8, **characterized in that** the allergens are immobilized on a glass and synthetic carrier, respectively, as the microarray chip.

11. The method according to any one of claims 1 to 8, **characterized in that** the allergens are immobilized on a silicon wafer as the microarray chip.

12. The method according to any one of claims 1 to 8, **characterized in that** the allergens are immobilized on a membrane as the microarray chip.

13. The method according to any one of claims 1 to 12, **characterized in that** the microarray chip is chemically modified, preferably by aminoreactive and carboxyreactive modification, respectively.

14. The method according to any one of claims 1 to 13, **characterized in that** the allergens are covalently bound to the microarray chip.

15. The method according to any one of claims 1 to 14, **characterized in that** the immunoglobulins are detected in blood serum as the sample.

16. The method according to claim 15, **characterized in that** the blood serum is diluted 1:1 - 1:15, preferably 1:5.

17. The method according to any one of claims 1 to 16, **characterized in that** the sample is incubated 1 min. to 24 hours, preferably 1 to 2 hours, with the allergens.

18. The method according to any one of claims 1 to 17, **characterized in that** the sample is incubated at a temperature between 0 and 60°C, preferably at 37°C, with the allergens.

19. The method according to any one of claims 1 to 18, **characterized in that** the bound immunoglobulins are detected with at least one labelled, specific anti-immunoglobulin antibody.

20. The method according to claim 19, **characterized in that** the bound immunoglobulins are detected with at least one fluorescence labelled specific anti-immunoglobulin antibody.

21. The method according to claim 19, **characterized in that** the bound immunoglobulins are detected with at least one radioactive labelled specific anti-immunoglobulin antibody.

22. The method according to any one of claims 1 to 21, **characterized in that** one or more indoor allergens are immobilized as allergens.

23. The method according to any one of claims 1 to 21, **characterized in that** one or more outdoor allergens are immobilized as allergens.

24. The method according to any one of claims 1 to 21, **characterized in that** one or more food allergens are immobilized as allergens.

25. The method according to any one of claims 1 to 21, **characterized in that** one or more venom allergens are immobilized as allergens.

26. The method according to any one of claims 1 to 21, **characterized in that** one or more auto-allergens are immobilized as allergens.

27. A method for in vitro diagnosis of allergies in a patient, **characterized in that** a serum sample is taken from the patient after which the sample is analysed for immunoglobulins which bind to allergens according to a method according to any one of claims 1 to 26, whereby a microarray chip is used on which at least 10, preferably at least 50, still preferred at least 90, different allergens are immobilized, after which a positive reaction between the sample and the immobilized allergens is diagnosed as an allergy.

28. Microarray chip on which one or more allergens are immobilized.

29. The microarray chip according to claim 28 **characterized in that** the allergens are immobilized on a 100 to 500 µm diameter, preferably 200-300 µm diameter, spot.

30. The microarray chip according to claim 28 or 29, **characterized in that** it is a glass carrier.

31. The microarray chip according to claim 28 or 29, **characterized in that** it is a synthetic carrier.

32. The microarray chip according to claim 28 or 29, **characterized in that** it is a silicon wafer.

33. The microarray chip according to claim 28 or 29, **characterized in that** it is a membrane.

34. The microarray chip according to any one of claims 28 to 33, **characterized in that** it is chemically modified, preferably by aminoreactive and carboxyreactive modification, respectively.

35. The microarray chip according to any one of claims 28 to 34, **characterized in that** the allergens are covalently bound to it.

36. Kit, **characterized in that** it comprises a microarray chip according to any one of claims 28 to 35 and a first reagent comprising at least one immunoglobulin detecting reagent, preferably an anti-immunoglobulin antibody, preferably in a known concentration, and possibly a second reagent as a positive sample comprising at least one immunoglobulin which binds to an allergen.

37. Kit according to claim 36 for carrying out a method according to any one of claims 1 to 27.
